# EUROPÄISCHE PATENTANMELDUNG

(11) **EP 4 470 574 A1**
(43) Veröffentlichungstag der Anmeldung: **04.12.2024**
(21) Anmeldenummer: 23176418.4
(22) Anmeldetag: 31.05.2023
(51) Int. Cl.: A61M 1/16, A61M 1/34, A61M 1/36, G16H 20/40, G16H 40/60

(54) **VORRICHTUNG UND VERFAHREN ZUR STEUERUNG EINES BLUTBEHANDLUNGSGERÄTS**

(71) Anmelder: Fresenius Medical Care Deutschland GmbH, 61352 Bad Homburg (DE)
(72) Erfinder: Dahsler, Michael, 61352 Bad Homburg (DE); Wagner, Philipp, 61352 Bad Homburg (DE); Irrgang, Tobias, 61352 Bad Homburg (DE)
(74) Vertreter: Ricker, Mathias

(57) **Zusammenfassung**

Ein Verfahren zur Steuerung eines Blutbehandlungsgeräts, insbesondere Dialysegeräts, das eine Steuereinrichtung und eine als Ein- und Ausgabeeinrichtung ausgebildete Komponente des Blutbehandlungsgeräts aufweist, und das dazu eingerichtet ist, gesteuert von der Steuereinrichtung, eine Blutbehandlung unter Verwendung zumindest eines Einwegartikels und zumindest eines Werts zumindest eines Betriebsparameters des Blutbehandlungsgeräts zumindest teilweise automatisiert durchzuführen, weist die folgenden Schritte auf:
Erfassen, insbesondere mittels der Ein- und Ausgabeeinrichtung, einer Zuordnungsvorschrift, in der zumindest eine Eigenschaft, insbesondere eine physikalische Eigenschaft, zumindest eines zur Blutbehandlung verwendbaren Einwegartikels zumindest einem Wert eines bei der Durchführung der Blutbehandlung verwendbaren zumindest einen Betriebsparameters zugeordnet ist, und
Steuern, mittels der Steuereinrichtung, zumindest einer Komponente, insbesondere der Ein- und Ausgabeeinrichtung, des Blutbehandlungsgeräts, basierend auf der Zuordnungsvorschrift.

## Beschreibung

Die vorliegende Erfindung betrifft ein Verfahren zur Steuerung eines Blutbehandlungsgeräts, insbesondere Dialysegeräts, sowie eine Vorrichtung, die dazu eingerichtet ist, das erfindungsgemäße Verfahren durchzuführen.

Im Rahmen der Durchführung einer (extrakorporalen) Blutbehandlung des Bluts eines Patienten mittels eines Blutbehandlungsgeräts werden für jede Durchführung einer Blutbehandlung mehrere Einwegartikel (engl.: disposables), wie etwa ein Dialysator, ein Blutschlauchsystem, eine venöse Nadel, oder ein oder mehrere, in entsprechenden Behältern aufgenommene, Dialysekonzentrate eingesetzt, die mit dem Blutbehandlungsgerät während der Blutbehandlung verbunden sind.

In der Regel können bei Blutbehandlungsgeräten Werte von Betriebsparameter des Blutbehandlungsgeräts, wie etwa ein Wert einer Förderrate einer Blutpumpe oder ein jeweiliger, einer jeweiligen Blutbehandlungsart zugeordneter Wert zur Durchführung der (jeweiligen) Blutbehandlung variabel und ohne Beschränkungen eingestellt werden.

Jedoch werden häufig je nach verwendeten Einwegartikel bestimmte Blutbehandlungsgerät-Funktionen bzw. bestimmte Blutbehandlungsarten und entsprechende Blutbehandlungsgerät-Einstellungen aus klinischer Sicht und/oder regulatorisch nicht empfohlen oder erlaubt.

Durch eine individuelle (manuelle) Eingabe der Betriebsparameter in Abhängigkeit von den verwendeten Einwegartikel unter Berücksichtigung aller Einwegartikel-bedingten Einschränkungen und/oder der Art der durchzuführenden Blutbehandlung am Blutbehandlungsgerät wird eine Fehleranfälligkeit bei der Bedienung bzw. der Einstellung der Betriebsparameter stark erhöht. Hieraus kann sich eine erhöhte Gefährdung für den Patienten und/oder ein erhöhter erforderlicher Zeitaufwand, insbesondere des medizinischen Personals und/oder des Patienten, für die Durchführung der Blutbehandlung ergeben.

Aus der EP 3 315 149 A1 ist ein Verfahren zur Eingabe von Geräte- bzw. Behandlungsparameter an Blutreinigungsgeräten bekannt, wobei Parameter zu einem verwendbaren Einwegartikel vor der Eingabe zugeordnet (registriert) werden. Bei der Eingabe eines so registrierten Einwegartikels werden entsprechende Parameter aus der Registrierung geladen. Die Einwegartikel-bedingten Einschränkungen werden bei der Registrierung anhand einer Zuweisung zwischen dem Einwegartikel und Parameter durch Empfehlungen bzw. Limitierungen umgesetzt. Hierbei ist jedoch nicht angegeben, wie Parametereinschränkungen nach der Vorregistrierung, insbesondere bei nachfolgenden Änderungen, die von der (Vor-)Registrierung abweichen, gehandhabt werden.

Aus der WO2022/238328 A1 ist ein Verfahren zur Parametereingabe an einem Dialysegerät bekannt, bei dem die Kompatibilität zwischen Parameter und IDs von Einwegartikel über einen Konsistenzabgleich (engl.: consistency procedure) sichergestellt wird. Hierbei wird insbesondere vorausgesetzt, dass eine eindeutige Zuweisung einer ID des Einwegartikels zu entsprechenden Parametereinschränkungen vorhanden ist.

Es ist eine Aufgabe der vorliegenden Erfindung, ein verbessertes Verfahren zur Steuerung eines Blutbehandlungsgeräts sowie eine verbesserte Vorrichtung zur Durchführung des erfindungsgemäßen Verfahren anzugeben.

Diese Aufgabe wird durch die Merkmale der unabhängigen Patentansprüche gelöst. Verschiedene Ausführungsformen und Weiterbildungen der Erfindung sind Gegenstand der Unteransprüche.

Ein erster Aspekt der Erfindung betrifft ein Verfahren, in einigen Ausführungen computerimplementiertes Verfahren, zur Steuerung eines Blutbehandlungsgeräts, in einigen Ausführungen Dialysegeräts, das eine Steuereinrichtung und eine als Ein- und Ausgabeeinrichtung ausgebildete Komponente des Blutbehandlungsgeräts aufweist, und das dazu eingerichtet ist, gesteuert von der Steuereinrichtung, eine Blutbehandlung unter Verwendung zumindest eines Einwegartikels und zumindest eines Werts zumindest eines Betriebsparameters des Blutbehandlungsgeräts zumindest teilweise automatisiert durchzuführen, wobei das Verfahren die folgenden Schritte aufweist: Erfassen, in einigen Ausführungen mittels der Ein- und Ausgabeeinrichtung, einer Zuordnungsvorschrift, in der zumindest eine Eigenschaft, in einigen Ausführungen eine physikalische Eigenschaft, zumindest eines zur Blutbehandlung verwendbaren Einwegartikels zumindest einem Wert eines bei der Durchführung der Blutbehandlung verwendbaren zumindest einen Betriebsparameters (des Blutbehandlungsgeräts) zugeordnet ist, und
Steuern, mittels der Steuereinrichtung, zumindest einer Komponente, in einigen Ausführungen der Ein- und Ausgabeeinrichtung, des Blutbehandlungsgeräts, basierend auf der Zuordnungsvorschrift.

Hierbei ist die Zuordnungsvorschrift in einigen Ausführungen derart gestaltet, dass ein jeweiliger Einwegartikel und ein oder mehrere Werte eines oder mehrerer Betriebsparameter für die Durchführung der Blutreinigung kompatibel sind, wenn die zumindest eine Eigenschaft des Einwegartikels dem zumindest einen Wert des zumindest einen Betriebsparameters (des Blutbehandlungsgeräts) in der Zuordnungsvorschrift zugeordnet ist.

Hierdurch kann in einigen Ausführungen verhindert werden, dass die Blutbehandlung mit einem Einwegartikel mit einer Eigenschaft durchgeführt wird, die nicht mit einem bei der Durchführung der Blutreinigung verwendeten Wert eines Betriebsparameters kompatibel ist, und somit die Sicherheit des Patienten erhöht und/oder der Zeitaufwand für die Durchführung der Blutbehandlung verringert werden.

Weiterhin können hierdurch Einwegartikel-abhängige Einstellungseinschränkungen des Blutbehandlungsgeräts auf einfache Weise bei der Durchführung der Blutbehandlung berücksichtigt werden. Insbesondere kann hierdurch bewirkt werden, dass Einwegartikel-abhängige Einstellungseinschränkungen kontinuierlich während der gesamten Nutzerinteraktion mit dem Blutbehandlungsgerät in die dynamische Nutzerführung eingehen.

Zudem wird dadurch, dass die Steuerung der zumindest einen Komponente des Blutbehandlungsgeräts auf einer Zuordnungsvorschrift basiert, in der Einwegartikel-Eigenschaften Betriebsparameter (des Blutbehandlungsgeräts) zugeordnet sind, eine Kompatibilitätsprüfung basierend auf den Einwegartikel-Eigenschaften ermöglicht, im Unterschied zu einer Kompatibilitätsprüfung basierend auf willkürlichen Einwegartikel-IDs, wodurch der kompatible Einsatz von unbekannten, das heißt nicht über ID-Code identifizierten, Einwegartikeln ermöglicht wird, und die Notwendigkeit ständiger Updates zur Erfassung neuer, (bisher) nicht registrierter, Einwegartikel entfällt.

Hierbei kann das Erfassen der Zuordnungsvorschrift beispielsweise durch ein Abrufen der Zuordnungsvorschrift aus einer Speichereinrichtung des Blutreinigungsgeräts, in der die Zuordnungsvorschrift gespeichert ist, oder aus einer externen Speichereinheit, beispielsweise einem Cloud-Speicher, in der die Zuordnungsvorschrift gespeichert ist, in einigen Ausführungen mittels einer entsprechenden Kommunikationsvorrichtung des Blutreinigungsgeräts, erfolgen.

In einigen Ausführungen weist das Verfahren ferner auf:
Erfassen zumindest eines, in einigen Ausführungen in einer Speichereinrichtung des Blutbehandlungsgeräts gespeicherten, voreingestellten Werts zumindest eines bei der Durchführung der Blutbehandlung von dem Blutbehandlungsgerät zu verwendenden Betriebsparameters des Blutbehandlungsgeräts, und/oder
Erfassen zumindest einer Eigenschaft, insbesondere einer physikalischen Eigenschaft, zumindest eines bei der Durchführung der Blutbehandlung zu verwendenden Einwegartikels, wobei das Steuern der zumindest einen Komponente des Blutbehandlungsgeräts ferner basierend auf dem zumindest einen voreingestellten Wert des zumindest einen bei der Durchführung der Blutbehandlung von dem Blutbehandlungsgerät zu verwendenden Betriebsparameters des Blutbehandlungsgeräts, und/oder auf der erfassten, zumindest einen Eigenschaft des zumindest einen bei der Durchführung der Blutbehandlung zu verwendenden Einwegartikels erfolgt.

Hierdurch kann in einigen Ausführungen die Sicherheit des Patienten weiter erhöht und/oder der Zeitaufwand für die Durchführung der Blutbehandlung weiter verringert werden.

In einigen Ausführungen wird der zumindest eine voreingestellte Wert des zumindest einen bei der Durchführung der Blutbehandlung von dem Blutbehandlungsgerät zu verwendenden Betriebsparameters des Blutbehandlungsgeräts erfasst, wobei
die zumindest eine Eigenschaft des bei der Durchführung der Blutbehandlung zumindest einen zu verwendenden Einwegartikels erfasst wird, und das Verfahren ferner aufweist:
Durchführen einer Prüfung, ob die erfasste, zumindest eine Eigenschaft des zumindest einen bei der Durchführung der Blutbehandlung zu verwendenden Einwegartikels in der Zuordnungsvorschrift dem zumindest einen voreingestellten Wert des bei der Durchführung der Blutbehandlung zu verwendenden zumindest einen Betriebsparameters zugeordnet ist oder nicht, wobei
das Steuern der zumindest einen Komponente des Blutbehandlungsgeräts ferner basierend auf einem Ergebnis der durchgeführten Prüfung erfolgt.

Hierdurch kann in einigen Ausführungen die Sicherheit des Patienten weiter erhöht und/oder der Zeitaufwand für die Durchführung der Blutbehandlung weiter verringert werden.

In einigen Ausführungen wird die Durchführung der Blutbehandlung mit dem Blutbehandlungsgerät mit dem erfassten zumindest einen voreingestellten Wert des zumindest einen bei der Durchführung der Blutbehandlung von dem Blutbehandlungsgerät zu verwendenden Betriebsparameters des Blutbehandlungsgeräts ermöglicht und/oder gestartet, wenn die erfasste, zumindest eine Eigenschaft des zumindest einen zu verwendenden Einwegartikels in der Zuordnungsvorschrift dem erfassten, zumindest einen voreingestellten Wert des zumindest einen bei der Durchführung der Blutbehandlung zu verwendenden Betriebsparameters zugeordnet ist, wobei
die Durchführung der Blutbehandlung mit dem Blutbehandlungsgerät mit dem erfassten zumindest einen voreingestellten Wert des zumindest einen bei der Durchführung der Blutbehandlung von dem Blutbehandlungsgerät zu verwendenden Betriebsparameters des Blutbehandlungsgeräts blockiert wird, wenn die erfasste, zumindest eine Eigenschaft des zumindest einen zu verwendenden Einwegartikels in der Zuordnungsvorschrift dem erfassten, zumindest einen voreingestellten Wert des zumindest einen bei der Durchführung der Blutbehandlung zu verwendenden Betriebsparameters nicht zugeordnet ist.

Hierdurch kann in einigen Ausführungen sichergestellt werden, dass die Blutbehandlung nicht mit einem Einwegartikel mit einer Eigenschaft durchgeführt wird, die nicht mit dem zumindest einen Wert des zumindest einen Betriebsparameters kompatibel ist.

Hierbei kann die Durchführung der Blutbehandlung mit dem Blutbehandlungsgerät ermöglicht und/oder gestartet werden, wenn eine Bestätigung einer mittels der Ein- und Ausgabeeinrichtung eingegebenen Eingabe der erfassten, zumindest einen Eigenschaft des zumindest einen bei der Durchführung der Blutbehandlung zu verwendenden Einwegartikels an der Ein- und Ausgabeeinrichtung erfasst wird, wobei
die Durchführung der Blutbehandlung mit dem Blutbehandlungsgerät blockiert wird, indem die Bestätigung der mittels der Ein- und Ausgabeeinrichtung eingegebenen Eingabe der erfassten, zumindest einen Eigenschaft des zumindest einen bei der Durchführung der Blutbehandlung zu verwendenden Einwegartikels an der Ein- und Ausgabeeinrichtung blockiert wird, und
optional zusätzlich eine optische und/oder akustische Warnmeldung, in einigen Ausführungen von der Steuereinrichtung, generiert und mittels der Ein- und Ausgabeeinrichtung ausgegeben wird, wenn die Durchführung der Blutbehandlung mit dem Blutbehandlungsgerät blockiert wird und/oder basierend auf der Zuordnungsvorschrift eine optische und/oder akustische Mitteilung mit einer Empfehlung zur Einstellung des zumindest einen bei der Durchführung der Blutbehandlung zu verwendenden Betriebsparameters, in einer Ausführung von der Steuereinrichtung, generiert wird und mittels der Ein- und Ausgabeeinrichtung ausgegeben wird.

Hierdurch kann in einigen Ausführungen eine korrekte Einstellung des zumindest einen Werts des zumindest einen Behandlungsparameters erleichtert werden.

In einigen Ausführungen wird der zumindest eine voreingestellte Wert des zumindest einen bei der Durchführung der Blutreinigung von dem Blutbehandlungsgerät zu verwendenden Betriebsparameters des Blutbehandlungsgeräts erfasst, wobei eine Eingabe, in einigen Ausführungen unter Verwendung der Ein- und Ausgabeeinrichtung, der zumindest einen Eigenschaft des zumindest einen bei der Durchführung der Blutbehandlung zu verwendenden Einwegartikels nur ermöglicht wird, wenn die zumindest eine Eigenschaft des zumindest einen Einwegartikels in der Zuordnungsvorschrift dem zumindest einen voreingestellten Wert des zumindest einen bei der Durchführung der Blutbehandlung von dem Blutbehandlungsgerät zu verwendenden Betriebsparameters des Blutbehandlungsgeräts zugeordnet ist, und die Durchführung der Blutbehandlung mit dem Blutbehandlungsgerät mit dem erfassten, zumindest einen voreingestellten Wert des zumindest einen bei der Durchführung der Blutbehandlung von dem Blutbehandlungsgerät zu verwendenden Betriebsparameters des Blutbehandlungsgeräts ermöglicht und/oder gestartet wird, wenn eine Eingabe der zumindest einen Eigenschaft des zumindest einen zu verwendenden Einwegartikels, die in der Zuordnungsvorschrift dem zumindest einen voreingestellten Wert des bei der Durchführung der Blutbehandlung zu verwendenden zumindest einen Betriebsparameters zugeordnet ist, erfasst wird.

Hierdurch kann in einigen Ausführungen eine korrekte Einstellung des zumindest einen Werts des zumindest einen Behandlungsparameters (weiter) erleichtert werden.

Hierbei kann in einigen Ausführungen die Funktion, dass die Eingabe der zumindest einen Eigenschaft des zumindest einen bei der Durchführung der Blutbehandlung zu verwendenden Einwegartikels nur ermöglicht wird, wenn die zumindest eine Eigenschaft des zumindest einen Einwegartikels in der Zuordnungsvorschrift dem zumindest einen voreingestellten Wert des zumindest einen bei der Durchführung der Blutbehandlung von dem Blutbehandlungsgerät zu verwendenden Betriebsparameters des Blutbehandlungsgeräts zugeordnet ist, dadurch verwirklicht werden, dass die Ein- und Ausgabeeinrichtung derart angesteuert wird, dass mittels der Ein- und Ausgabeeinrichtung nur solche Eigenschaften des Einwegartikels zur Auswahl und Eingabe vorgeschlagen werden, beispielsweise durch eine entsprechende Darstellung auf einer grafischen Benutzeroberfläche eines Bildschirms, die dem zumindest einen voreingestellten Wert des zumindest einen bei der Durchführung der Blutbehandlung von dem Blutbehandlungsgerät zu verwendenden Betriebsparameters des Blutbehandlungsgeräts zugeordnet sind.

In einigen Ausführungen wird die zumindest eine Eigenschaft des zumindest einen bei der Durchführung der Blutbehandlung zu verwendenden Einwegartikels erfasst, wobei zumindest ein Wert des zumindest einen bei der Durchführung der Blutbehandlung von dem Blutbehandlungsgerät zu verwendenden Betriebsparameters erfasst wird,
die Durchführung der Blutbehandlung mit dem Blutbehandlungsgerät oder eine Fortsetzung einer momentan mit dem Blutbehandlungsgerät durchgeführten Blutbehandlung mit dem erfassten zumindest einen Wert des zumindest einen bei der Durchführung der Blutbehandlung von dem Blutbehandlungsgerät zu verwendenden Betriebsparameters des Blutbehandlungsgeräts ermöglicht und/oder gestartet wird, wenn die erfasste, zumindest eine Eigenschaft des zumindest einen zu verwendenden Einwegartikels in der Zuordnungsvorschrift dem erfassten, zumindest einen Wert des zumindest einen bei der Durchführung der Blutbehandlung von dem Blutbehandlungsgerät zu verwendenden Betriebsparameters zugeordnet ist, und
die Durchführung der Blutbehandlung mit dem Blutbehandlungsgerät oder die Fortsetzung der momentan mit dem Blutbehandlungsgerät durchgeführten Blutbehandlung mit dem erfassten zumindest einen Wert des zumindest einen bei der Durchführung der Blutbehandlung von dem Blutbehandlungsgerät zu verwendenden Betriebsparameters des Blutbehandlungsgeräts blockiert wird, wenn die erfasste, zumindest eine Eigenschaft des zumindest einen bei der Durchführung der Blutbehandlung zu verwendenden Einwegartikels in der Zuordnungsvorschrift dem erfassten, zumindest einen Wert des bei der Durchführung der Blutbehandlung zu verwendenden zumindest einen Betriebsparameters nicht zugeordnet ist.

In einigen Ausführungen wird die Durchführung der Blutbehandlung mit dem Blutbehandlungsgerät oder die Fortsetzung der momentan mit dem Blutbehandlungsgerät durchgeführten Blutbehandlung ermöglicht und/oder gestartet, wenn eine Bestätigung einer mittels der Ein- und Ausgabeeinrichtung eingegebenen Eingabe der erfassten, zumindest einen Eigenschaft des zumindest einen bei der Durchführung der Blutbehandlung zu verwendenden Einwegartikels an der Ein- und Ausgabeeinrichtung erfasst wird, wobei
die Durchführung der Blutbehandlung mit dem Blutbehandlungsgerät oder die Fortsetzung der momentan mit dem Blutbehandlungsgerät durchgeführten Blutbehandlung blockiert wird, indem die Bestätigung der mittels der Ein- und Ausgabeeinrichtung eingegebenen Eingabe der erfassten, zumindest einen Eigenschaft des zumindest einen zu verwendenden Einwegartikels an der Ein- und Ausgabeeinrichtung blockiert wird.

In einigen Ausführungen beinhaltet das Erfassen der zumindest einen Eigenschaft des zumindest einen bei der Durchführung der Blutbehandlung zu verwendenden Einwegartikels ein Erfassen einer Eingabe der zumindest einen Eigenschaft des zumindest einen bei der Durchführung der Blutbehandlung zu verwendenden Einwegartikels mittels einer grafischen Benutzeroberfläche der Ein- und Ausgabeeinrichtung und/oder ein Herunterladen der zumindest einen Eigenschaft des zumindest einen bei der Durchführung der Blutbehandlung zu verwendenden Einwegartikels mittels der Ein- und Ausgabeeinrichtung von einem externen Gerät und/oder ein Scannen eines dem zumindest einen bei der Durchführung der Blutbehandlung zu verwendenden Einwegartikel zugeordneten Codes mittels einer Scan-Einrichtung der Ein- und Ausgabeeinrichtung, wobei die Steuereinrichtung die zumindest eine Eigenschaft des zumindest einen bei der Durchführung der Blutbehandlung zu verwendenden Einwegartikels basierend auf dem gescannten Code erfasst.

In einigen Ausführungen sind jeweilige Werte des zumindest einen Werts eines Betriebsparameters des zumindest einen Betriebsparameters jeweiligen unterschiedlichen Arten der Blutbehandlung zugeordnet. Hierbei können die unterschiedlichen Arten der Blutbehandlung eine Hämodialyse, eine Hämofiltration, eine Hämodiafiltration, und eine pädiatrische Blutbehandlung bzw. Dialyse beinhalten.

In einigen Ausführungen beinhaltet der zumindest eine Einwegartikel einen Dialysator, ein Blutschlauchsystem, eine venöse Nadel, und ein oder mehrere Dialysekonzentrate, welche vorzugsweise in einem Behälter aufgenommen sind.

Hierbei kann die zumindest eine Eigenschaft des Dialysators einen Ultrafiltrationskoeffizienten des Dialysators, ein Volumen des Dialysators, und einen Längswiderstand des Dialysators, die zumindest eine Eigenschaft des Blutschlauchsystems ein Volumen des Blutschlauchsystems, einen Flusswiderstand des Blutschlauchsystems, und eine Eignung des Blutschlauchsystems für Pre-, Post- und/oder Mixed-Substitution im Rahmen eines Hämodiafiltration-Substitutionsverfahrens, und die zumindest eine Eigenschaft der venösen Nadel einen Flusswiderstand der venösen Nadel beinhalten.

In einigen Ausführungen weist das Blutbehandlungsgerät als eine weitere Komponente eine Blutpumpe auf, die während der Durchführung der Blutbehandlung über das Blutschlauchsystem mit dem Dialysator und der venösen Nadel fluidisch verbunden sein kann, wobei der zumindest eine Wert zumindest eines Betriebsparameters des Blutbehandlungsgeräts einen Wert einer Förderrate der Blutpumpe beinhalten kann.

Mittels entsprechender Zuordnungen in der Zuordnungsvorschrift (und gegebenenfalls zusätzlich generierter und mittels der Ein- und Ausgabeeinrichtung ausgegebener Meldungen) können beispielsweise:
- ein Füllverfahren und ein Entleerungsverfahren des Blutschlauchsystems, welche Teile der von dem Blutbehandlungsgerät durchgeführten Blutbehandlung sind, anhand des Ultrafiltrationskoeffizienten zugelassen, eingeschränkt oder gesperrt werden,
- das Füllverfahren und das Entleerungsverfahren des Blutschlauchsystems an das Volumen des Dialysators und des Blutschlauchsystems angepasst werden,
- Pediatrische/Low Volume Blutbehandlungen anhand des Volumens des Dialysators und des Blutschlauchsystems zugelassen, eingeschränkt oder gesperrt werden,
- Hämodiafiltration-Substitutionsverfahren anhand dessen zugelassen werden, ob das Blutschlauchsystem für Pre, Post und/oder Mixed Substitution ausgelegt ist,
- die Blutpumpenrate anhand des Längswiderstandes des Dialysators und des Flusswiderstandes des Blutschlauchsystems und der venösen Nadel eingeschränkt werden,
- nicht kompatible/ungünstige Kombinationen von Dialysekonzentraten ausgeschlossen werden oder mittels der Ausgabe einer entsprechenden Meldung mittels der Ein- und Ausgabeeinrichtung auf mögliche Folgen hingewiesen werden:
   o etwa im Falle von zu verwendenden Kombinationen unterschiedlicher Dialysekonzentrate, bei denen es aufgrund eines hohen MgCl₂- und/oder CaCl-Gehalts zu verfrühten Einschränkungen aufgrund von Kalkablagerungen im Blutbehandlungsgerät kommen kann, kann eine entsprechende Meldung ausgegeben werden,
   ∘ im Falle, dass Bibag/Trockenbicarbonat mit Säurekonzentrat verwendet werden, welches nur mit flüssigem Bicarbonat-Konzentrat verwendet werden kann, in dem NaCl enthalten ist, kann eine entsprechende Meldung ausgegeben werden,
   ∘ im Falle, dass zu verwendende Dialysekonzentrate aufgrund ihrer Inhaltstoffe nicht zur Anmischung eines verschriebenen Dialysats verwendet werden können, kann eine entsprechende Meldung ausgegeben werden, beispielsweise wenn ein Kalium-Gehalt nicht mit einer Verschreibung eines zu behandelnden Patienten übereinstimmt,
   o basierend auf einem Volumen des Behälters des Dialysekonzentrats und einer Füllmenge des Trockendialysekonzentrats kann eine Meldung darüber ausgegeben werden, ob das angeschlossene Dialysekonzentrat für eine Dauer einer gewählten Behandlungsart ausreichend ist.

Gemäß einem zweiten Aspekt der Erfindung ist eine Vorrichtung dazu eingerichtet, ein im vorhergehenden beschriebenes Verfahren zur Steuerung eines Blutbehandlungsgeräts, insbesondere Dialysegeräts, durchzuführen.

Die in Bezug auf den ersten Aspekt der Erfindung und dessen vorteilhafte Ausgestaltung beschriebenen Merkmale und Vorteile gelten, zumindest wo technisch sinnvoll auch für den zweiten Aspekt der Erfindung und dessen vorteilhafte Ausgestaltung sowie umgekehrt.

Weitere Vorteile, Merkmale und Anwendungsmöglichkeiten der vorliegenden Erfindung ergeben sich aus der nachfolgenden Beschreibung in Zusammenhang mit den Figuren, in denen durchgängig dieselben Bezugszeichen für dieselben oder einander entsprechende Elemente der Erfindung verwendet werden. Es zeigen, wenigstens teilweise schematisch:
- Fig. 1: ein Blutbehandlungsgerät, welches mittels einem erfindungsgemäßen Verfahren zur Steuerung eines Blutbehandlungsgerät gesteuert werden kann, und welches die erfindungsgemäße Vorrichtung zur Steuerung eines Blutbehandlungsgeräts aufweist, und
- Fig. 2: ein Flussdiagramm zur Veranschaulichung eines erfindungsgemäßen Verfahren zur Steuerung eines Blutbehandlungsgerät gemäß einer Ausführung.

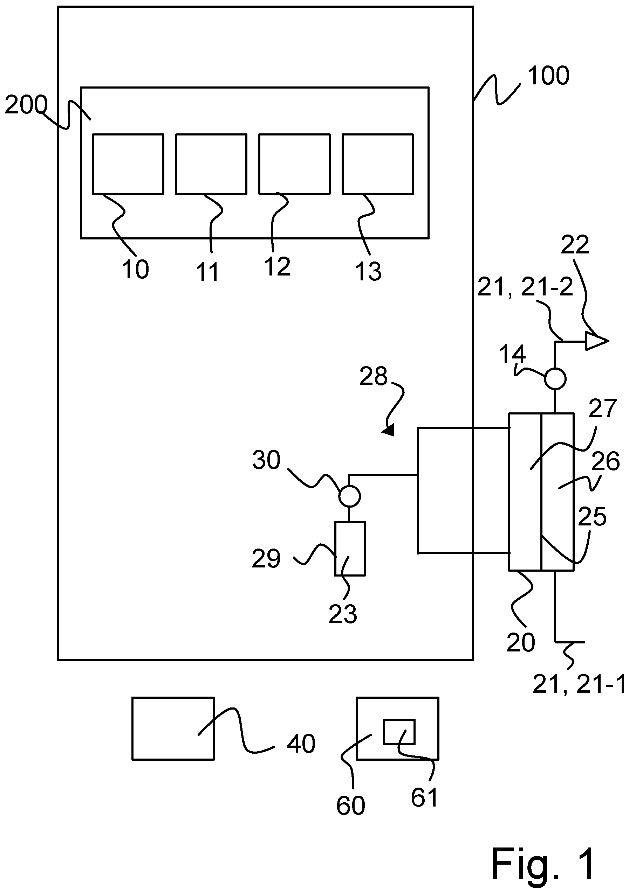

Fig. 1 zeigt ein Blutbehandlungsgerät 100, in einigen Ausführungen Dialysegerät, welches mittels einem erfindungsgemäßen Verfahren zur Steuerung eines Blutbehandlungsgerät 100 gesteuert werden kann, und welches die erfindungsgemäße Vorrichtung 200 zur Steuerung eines Blutbehandlungsgeräts 100 aufweist.

Die Vorrichtung 200 weist eine Steuereinrichtung 10 sowie eine Ein- und Ausgabeeinrichtung 11, eine Kommunikationsvorrichtung 12 und eine Speichereinrichtung 13 auf, wobei die Steuereinrichtung 10 mit der Ein- und Ausgabeeinrichtung 11, der Kommunikationsvorrichtung 12 und der Speichereinrichtung 13 in Kommunikationsverbindung steht und Daten mit diesen austauschen kann, und dazu eingerichtet ist, das Blutbehandlungsgerät 100 zu steuern.

Das Blutbehandlungsgerät 100 weist ferner einen Dialysator 20 auf, welcher durch eine semipermeable Membran 25 in eine Blutkammer 26 und eine von Dialyseflüssigkeit durchflossene Dialyseflüssigkeitskammer 27 unterteilt ist. Die Blutkammer 26 ist ein Teil eines extrakorporalen Blutkreislaufs, während die Dialyseflüssigkeitskammer 27 Teil eines Dialyseflüssigkeitssystem 28 ist, welches hier nur angedeutet ist, und welches einen Behälter 29 aufweist, in dem Dialysekonzentrat 23 aufgenommen ist, das mittels einer Dialyseflüssigkeitspumpe 30 in die Dialyseflüssigkeitskammer 27 gepumpt wird.

Der extrakorporale Blutkreislauf umfasst ferner ein Blutschlauchsystem 21 mit einer arteriellen Blutleitung 21-1, die zu der Blutkammer 26 führt, und einer venösen Blutleitung 21-2, die von der Blutkammer 26 des Dialysators 20 wegführt und an deren Ende eine venöse Nadel 22 vorgesehen ist.

Hierbei sind der Dialysator 20, das Blutschlauchsystem 21, die venöse Nadel 22 und das Dialysekonzentrat 23 Einwegartikel, die nur für eine einzige Blutbehandlung verwendet werden können.

Da insoweit der Aufbau und die Funktionsweise eines derartigen Blutbehandlungsgeräts 100 bekannt sind, kann auf eine genauere Beschreibung desselben an dieser Stelle verzichtet werden.

Das Blutbehandlungsgerät 100 ist dazu eingerichtet, gesteuert von der Steuereinrichtung 10, eine Blutbehandlung unter Verwendung zumindest eines der Einwegartikel 20, 21, 22, 23, insbesondere aller der Einwegartikel 20, 21, 22, 23, und zumindest eines Werts zumindest eines Betriebsparameters des Blutbehandlungsgeräts 100 zumindest teilweise automatisiert durchzuführen.

Ferner ist das Blutbehandlungsgerät 100 dazu eingerichtet, in einigen Ausführungen mittels der Ein- und Ausgabeeinrichtung 11, eine Zuordnungsvorschrift zu erfassen, in der zumindest eine Eigenschaft, in einigen Ausführungen eine physikalische Eigenschaft, zumindest eines Einwegartikels 20, 21, 22, 23 der zur Blutbehandlung verwendbaren Einwegartikel 20, 21, 22, 23 zumindest einem Wert eines bei der Durchführung der Blutbehandlung verwendbaren zumindest einen Betriebsparameters (des Blutbehandlungsgeräts) zugeordnet ist.

Hierbei ist die Steuereinrichtung 10 dazu eingerichtet, zumindest eine Komponente 11, 14, in einigen Ausführungen die Ein- und Ausgabeeinrichtung 11, des Blutbehandlungsgeräts 100, basierend auf der Zuordnungsvorschrift zu steuern.

Hierbei ist die Zuordnungsvorschrift in einigen Ausführungen derart gestaltet, dass ein jeweiliger Einwegartikel 20, 21, 22, 23 und ein oder mehrere Werte eines oder mehrerer Betriebsparameter für die Durchführung der Blutreinigung kompatibel sind, wenn die zumindest eine Eigenschaft des Einwegartikels 20, 21, 22, 23 dem zumindest einen Wert des zumindest einen Betriebsparameters in der Zuordnungsvorschrift zugeordnet ist.

Das Erfassen der Zuordnungsvorschrift kann beispielsweise durch ein Abrufen der Zuordnungsvorschrift aus der Speichereinrichtung 13 des Blutreinigungsgeräts 100, in der die Zuordnungsvorschrift gespeichert ist, oder aus einer externen Speichereinheit 60, beispielsweise einem Cloud-Speicher, in der die Zuordnungsvorschrift gespeichert ist, in einigen Ausführungen mittels der Kommunikationsvorrichtung 12 des Blutreinigungsgeräts 100, die dazu eingerichtet ist, mit einer Kommunikationseinrichtung 61 der externen Speichereinheit 60 drahtlos zu kommunizieren, erfolgen.

Hierbei kann der Betriebsparameter des Blutbehandlungsgeräts 100 beispielsweise eine Förderrate der Blutpumpe 14 sein. Weiterhin können hierbei jeweilige Werte des zumindest einen Werts eines Betriebsparameters des zumindest einen Betriebsparameters jeweiligen unterschiedlichen Arten der Blutbehandlung zugeordnet sein.

Die zumindest eine Eigenschaft des Dialysators 20 kann einen Ultrafiltrationskoeffizienten des Dialysators 20, ein Volumen des Dialysators 20, und einen Längswiderstand des Dialysators 20 beinhalten.

Die zumindest eine Eigenschaft des Blutschlauchsystems 21 kann ein Volumen des Blutschlauchsystems 21, einen Flusswiderstand des Blutschlauchsystems 21, und eine Eignung des Blutschlauchsystems 21 für Pre-, Post- und/oder Mixed-Substitution im Rahmen eines Hämodiafiltration-Substitutionsverfahrens beinhalten.

Das Behandlungsgerät 100 ist in einigen Ausführungen ferner dazu eingerichtet, einen, beispielsweise in der Speichereinrichtung 13 gespeicherten, voreingestellten Wert zumindest eines bei der Durchführung der Blutbehandlung von dem Blutbehandlungsgerät 100 zu verwendenden Betriebsparameters des Blutbehandlungsgeräts 100 zu erfassen.

Alternativ oder zusätzlich dazu kann das Behandlungsgerät 100 dazu eingerichtet sein, zumindest eine Eigenschaft, insbesondere eine physikalische Eigenschaft, zumindest eines bei der Durchführung der Blutbehandlung zu verwendenden Einwegartikels 20, 21, 22, 23 zu erfassen.

Hierbei kann das Steuern der zumindest einen Komponente 11, 14 des Blutbehandlungsgeräts 100 ferner basierend auf dem zumindest einen voreingestellten Wert des zumindest einen bei der Durchführung der Blutbehandlung von dem Blutbehandlungsgerät 100 zu verwendenden Betriebsparameters des Blutbehandlungsgeräts 100, und/oder auf der erfassten, zumindest einen Eigenschaft des zumindest einen bei der Durchführung der Blutbehandlung zu verwendenden Einwegartikels 20, 21, 22, 23 erfolgen.

Das Erfassen der zumindest einen Eigenschaft des zumindest einen bei der Durchführung der Blutbehandlung zu verwendenden Einwegartikels 20, 21, 22, 23 kann ein Erfassen einer, von einem Benutzer durchgeführten, Eingabe der zumindest einen Eigenschaft des zumindest einen bei der Durchführung der Blutbehandlung zu verwendenden Einwegartikels 20, 21, 22, 23 mittels einer grafischen Benutzeroberfläche der Ein- und Ausgabeeinrichtung 11 beinhalten.

Zusätzlich oder alternativ dazu kann das Erfassen der zumindest einen Eigenschaft des zumindest einen bei der Durchführung der Blutbehandlung zu verwendenden Einwegartikels 20, 21, 22, 23 ein Herunterladen der zumindest einen Eigenschaft des zumindest einen bei der Durchführung der Blutbehandlung zu verwendenden Einwegartikels 20, 21, 22, 23 mittels der Ein- und Ausgabeeinrichtung 11 von einem externen Gerät 40 beinhalten.

Weiterhin zusätzlich oder alternativ kann das Erfassen der zumindest einen Eigenschaft des zumindest einen bei der Durchführung der Blutbehandlung zu verwendenden Einwegartikels 20, 21, 22, 23 ein Scannen eines dem zumindest einen bei der Durchführung der Blutbehandlung zu verwendenden Einwegartikel zugeordneten Codes mittels einer Scan-Einrichtung der Ein- und Ausgabeeinrichtung 11 beinhalten, wobei die Steuereinrichtung 10 dazu eingerichtet ist, die zumindest eine Eigenschaft des zumindest einen bei der Durchführung der Blutbehandlung zu verwendenden Einwegartikels 20, 21, 22, 23 basierend auf dem gescannten Code zu erfassen.

Wenn der zumindest eine voreingestellte Wert des zumindest einen bei der Durchführung der Blutbehandlung von dem Blutbehandlungsgerät 100 zu verwendenden Betriebsparameters des Blutbehandlungsgeräts 100 erfasst wird, und die zumindest eine Eigenschaft des bei der Durchführung der Blutbehandlung zumindest einen zu verwendenden Einwegartikels 20, 21, 22, 23 erfasst wird, kann in einigen Ausführungen das Blutbehandlungsgerät 100, insbesondere die Steuereinrichtung 10, dazu eingerichtet sein, eine Prüfung durchzuführen, ob die erfasste, zumindest eine Eigenschaft des zumindest einen bei der Durchführung der Blutbehandlung zu verwendenden Einwegartikels 20, 21, 22, 23 in der Zuordnungsvorschrift dem zumindest einen voreingestellten Wert des bei der Durchführung der Blutbehandlung zu verwendenden zumindest einen Betriebsparameters zugeordnet ist oder nicht.

In diesem Fall kann das Steuern der zumindest einen Komponente 11, 14 des Blutbehandlungsgeräts 100 ferner basierend auf einem Ergebnis der durchgeführten Prüfung erfolgen.

Des Weiteren kann in diesem Fall die Steuereinrichtung 10 dazu eingerichtet sein, die Durchführung der Blutbehandlung mit dem Blutbehandlungsgerät 100 mit dem erfassten zumindest einen voreingestellten Wert des zumindest einen bei der Durchführung der Blutbehandlung von dem Blutbehandlungsgerät 100 zu verwendenden Betriebsparameters des Blutbehandlungsgeräts 100 zu ermöglichen und/oder zu starten, wenn die erfasste, zumindest eine Eigenschaft des zumindest einen zu verwendenden Einwegartikels 20, 21, 22, 23 in der Zuordnungsvorschrift dem erfassten, zumindest einen voreingestellten Wert des zumindest einen bei der Durchführung der Blutbehandlung zu verwendenden Betriebsparameters zugeordnet ist, und die Durchführung der Blutbehandlung mit dem Blutbehandlungsgerät 100 mit dem erfassten zumindest einen voreingestellten Wert des zumindest einen bei der Durchführung der Blutbehandlung von dem Blutbehandlungsgerät 100 zu verwendenden Betriebsparameters des Blutbehandlungsgeräts 100 zu blockieren, wenn die erfasste, zumindest eine Eigenschaft des zumindest einen zu verwendenden Einwegartikels 20, 21, 22, 23 in der Zuordnungsvorschrift dem erfassten, zumindest einen voreingestellten Wert des zumindest einen bei der Durchführung der Blutbehandlung zu verwendenden Betriebsparameters nicht zugeordnet ist.

Insbesondere kann in diesem Fall die Steuereinrichtung 10 dazu eingerichtet sein, die Durchführung der Blutbehandlung mit dem Blutbehandlungsgerät 100 zu ermöglichen und/oder zu starten, wenn eine Bestätigung einer mittels der Ein- und Ausgabeeinrichtung 11 eingegebenen Eingabe der erfassten, zumindest einen Eigenschaft des zumindest einen bei der Durchführung der Blutbehandlung zu verwendenden Einwegartikels 20, 21, 22, 23 an der Ein- und Ausgabeeinrichtung 11 erfasst wird, und die Durchführung der Blutbehandlung mit dem Blutbehandlungsgerät 100 zu blockieren, indem die Bestätigung der mittels der Ein- und Ausgabeeinrichtung 11 eingegebenen Eingabe der erfassten, zumindest einen Eigenschaft des zumindest einen bei der Durchführung der Blutbehandlung zu verwendenden Einwegartikels 20, 21, 22, 23 an der Ein- und Ausgabeeinrichtung 11 blockiert wird.

Optional kann hierbei das Blutbehandlungsgerät 100 dazu eingerichtet sein, zusätzlich eine optische und/oder akustische Warnmeldung, insbesondere mittels der Steuereinrichtung 10, zu generieren, und mittels der Ein- und Ausgabeeinrichtung 11 auszugeben, wenn die Durchführung der Blutbehandlung mit dem Blutbehandlungsgerät 100 blockiert wird und/oder basierend auf der Zuordnungsvorschrift eine optische und/oder akustische Mitteilung mit einer Empfehlung zur Einstellung des zumindest einen bei der Durchführung der Blutbehandlung zu verwendenden Betriebsparameters, insbesondere mittels der Steuereinrichtung 10, zu generieren, und mittels der Ein- und Ausgabeeinrichtung 11 auszugeben.

In einigen Ausführungen ist das Blutbehandlungsgerät 100 dazu eingerichtet, den zumindest einen voreingestellten Wert des zumindest einen bei der Durchführung der Blutreinigung von dem Blutbehandlungsgerät 100 zu verwendenden Betriebsparameters des Blutbehandlungsgeräts 100 zu erfassen, und eine Eingabe, insbesondere unter Verwendung der Ein- und Ausgabeeinrichtung 11, der zumindest einen Eigenschaft des zumindest einen bei der Durchführung der Blutbehandlung zu verwendenden Einwegartikels 20, 21, 22, 23 nur zu ermöglichen, wenn die zumindest eine Eigenschaft des zumindest einen Einwegartikels 20, 21, 22, 23 in der Zuordnungsvorschrift dem zumindest einen voreingestellten Wert des zumindest einen bei der Durchführung der Blutbehandlung von dem Blutbehandlungsgerät 100 zu verwendenden Betriebsparameters des Blutbehandlungsgeräts 100 zugeordnet ist.

Hierbei ist die Steuereinrichtung 10 bevorzugt dazu eingerichtet, die Durchführung der Blutbehandlung mit dem Blutbehandlungsgerät 100 mit dem erfassten, zumindest einen voreingestellten Wert des zumindest einen bei der Durchführung der Blutbehandlung von dem Blutbehandlungsgerät 100 zu verwendenden Betriebsparameters des Blutbehandlungsgeräts 100 zu ermöglichen und/oder zu starten, wenn eine Eingabe der zumindest einen Eigenschaft des zumindest einen zu verwendenden Einwegartikels 20, 21, 22, 23, die in der Zuordnungsvorschrift dem zumindest einen voreingestellten Wert des bei der Durchführung der Blutbehandlung zu verwendenden zumindest einen Betriebsparameters zugeordnet ist, erfasst wird.

In einigen Ausführungen ist die Steuereinrichtung 10 dazu eingerichtet, die zumindest eine Eigenschaft des zumindest einen bei der Durchführung der Blutbehandlung zu verwendenden Einwegartikels 20, 21, 22, 23 zu erfassen, und zumindest einen Wert des zumindest einen bei der Durchführung der Blutbehandlung von dem Blutbehandlungsgerät 100 zu verwendenden Betriebsparameters zu erfassen.

In diesem Fall kann die Steuereinrichtung 10 dazu eingerichtet sein, die Durchführung der Blutbehandlung mit dem Blutbehandlungsgerät 100 oder eine Fortsetzung einer momentan mit dem Blutbehandlungsgerät 100 durchgeführten Blutbehandlung mit dem erfassten zumindest einen Wert des zumindest einen bei der Durchführung der Blutbehandlung von dem Blutbehandlungsgerät 100 zu verwendenden Betriebsparameters des Blutbehandlungsgeräts 100 zu ermöglichen und/oder zu starten, wenn die erfasste, zumindest eine Eigenschaft des zumindest einen zu verwendenden Einwegartikels 20, 21, 22, 23 in der Zuordnungsvorschrift dem erfassten, zumindest einen Wert des zumindest einen bei der Durchführung der Blutbehandlung von dem Blutbehandlungsgerät 100 zu verwendenden Betriebsparameters zugeordnet ist.

Weiterhin kann in diesem Fall die Steuereinrichtung 10 dazu eingerichtet sein, die Durchführung der Blutbehandlung mit dem Blutbehandlungsgerät 100 oder die Fortsetzung der momentan mit dem Blutbehandlungsgerät 100 durchgeführten Blutbehandlung mit dem erfassten zumindest einen Wert des zumindest einen bei der Durchführung der Blutbehandlung von dem Blutbehandlungsgerät 100 zu verwendenden Betriebsparameters des Blutbehandlungsgeräts 100 zu blockieren, wenn die erfasste, zumindest eine Eigenschaft des zumindest einen bei der Durchführung der Blutbehandlung zu verwendenden Einwegartikels 20, 21, 22, 23 in der Zuordnungsvorschrift dem erfassten, zumindest einen Wert des bei der Durchführung der Blutbehandlung zu verwendenden zumindest einen Betriebsparameters nicht zugeordnet ist.

Hierbei kann die Steuereinrichtung 10 in einigen Ausführungen dazu eingerichtet sein, die Durchführung der Blutbehandlung mit dem Blutbehandlungsgerät 100 oder die Fortsetzung der momentan mit dem Blutbehandlungsgerät 100 durchgeführten Blutbehandlung zu ermöglichen und/oder zu starten, wenn eine Bestätigung einer mittels der Ein- und Ausgabeeinrichtung 11 eingegebenen Eingabe der erfassten, zumindest einen Eigenschaft des zumindest einen bei der Durchführung der Blutbehandlung zu verwendenden Einwegartikels 20, 21, 22, 23 an der Ein- und Ausgabeeinrichtung 11 erfasst wird.

Außerdem kann hierbei die Steuereinrichtung 10 dazu eingerichtet sein, die Durchführung der Blutbehandlung mit dem Blutbehandlungsgerät 100 oder die Fortsetzung der momentan mit dem Blutbehandlungsgerät 100 durchgeführten Blutbehandlung zu blockieren, indem sie die Bestätigung der mittels der Ein- und Ausgabeeinrichtung 11 eingegebenen Eingabe der erfassten, zumindest einen Eigenschaft des zumindest einen zu verwendenden Einwegartikels 20, 21, 22, 23 an der Ein- und Ausgabeeinrichtung blockiert.

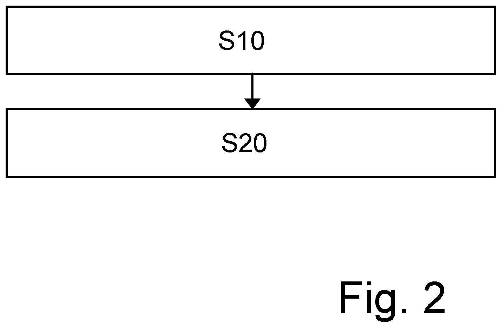

Fig. 2 zeigt ein Flussdiagramm zur Veranschaulichung eines erfindungsgemäßen Verfahren zur Steuerung eines Blutbehandlungsgerät 100, das eine Steuereinrichtung 10 und eine als Ein- und Ausgabeeinrichtung 11 ausgebildete Komponente des Blutbehandlungsgeräts 100 aufweist, und das dazu eingerichtet ist, gesteuert von der Steuereinrichtung 10, eine Blutbehandlung unter Verwendung zumindest eines Einwegartikels 20, 21, 22, 23 und zumindest eines Werts zumindest eines Betriebsparameters des Blutbehandlungsgeräts 100 zumindest teilweise automatisiert durchzuführen.

In einem Schritt S10 des Verfahren erfolgt ein Erfassen, in einigen Ausführungen mittels der Ein- und Ausgabeeinrichtung 11, einer Zuordnungsvorschrift, in der zumindest eine Eigenschaft, in einigen Ausführungen eine physikalische Eigenschaft, zumindest eines zur Blutbehandlung verwendbaren Einwegartikels 20, 21, 22, 23 zumindest einem Wert eines bei der Durchführung der Blutbehandlung verwendbaren zumindest einen Betriebsparameters (des Blutbehandlungsgeräts) zugeordnet ist.

In einem Schritt S20 des Verfahrens erfolgt ein Steuern, mittels der Steuereinrichtung 10, zumindest einer Komponente 11, 14, in einigen Ausführungen der Ein- und Ausgabeeinrichtung 11, des Blutbehandlungsgeräts 100, basierend auf der Zuordnungsvorschrift.

In einigen Ausführungen kann das Verfahren ferner ein Erfassen zumindest eines, in einigen Ausführungen in einer Speichereinrichtung 12 des Blutbehandlungsgeräts 100 gespeicherten, voreingestellten Werts zumindest eines bei der Durchführung der Blutbehandlung von dem Blutbehandlungsgerät 100 zu verwendenden Betriebsparameters des Blutbehandlungsgeräts 100 und/oder ein Erfassen zumindest einer Eigenschaft, in einigen Ausführungen einer physikalischen Eigenschaft, zumindest eines bei der Durchführung der Blutbehandlung zu verwendenden Einwegartikels 20, 21, 22, 23 aufweisen, wobei das Steuern der zumindest einen Komponente 11, 14 des Blutbehandlungsgeräts 100 ferner basierend auf dem zumindest einen voreingestellten Wert des zumindest einen bei der Durchführung der Blutbehandlung von dem Blutbehandlungsgerät 100 zu verwendenden Betriebsparameters des Blutbehandlungsgeräts 100 und/oder auf der erfassten, zumindest einen Eigenschaft des zumindest einen bei der Durchführung der Blutbehandlung zu verwendenden Einwegartikels 20, 21, 22, 23 erfolgt.

Hierbei kann der zumindest eine voreingestellte Wert des zumindest einen bei der Durchführung der Blutbehandlung von dem Blutbehandlungsgerät 100 zu verwendenden Betriebsparameters des Blutbehandlungsgeräts 100 erfasst werden, und die zumindest eine Eigenschaft des bei der Durchführung der Blutbehandlung zumindest einen zu verwendenden Einwegartikels 20, 21, 22, 23 erfasst werden, wobei das Verfahren ferner ein Durchführen einer Prüfung, ob die erfasste, zumindest eine Eigenschaft des zumindest einen bei der Durchführung der Blutbehandlung zu verwendenden Einwegartikels 20, 21, 22, 23 in der Zuordnungsvorschrift dem zumindest einen voreingestellten Wert des bei der Durchführung der Blutbehandlung zu verwendenden zumindest einen Betriebsparameters zugeordnet ist oder nicht aufweist, und das Steuern der zumindest einen Komponente 11, 14 des Blutbehandlungsgeräts 100 ferner basierend auf einem Ergebnis der durchgeführten Prüfung erfolgt.

In einigen Ausführungen kann die Durchführung der Blutbehandlung mit dem Blutbehandlungsgerät 100 mit dem erfassten zumindest einen voreingestellten Wert des zumindest einen bei der Durchführung der Blutbehandlung von dem Blutbehandlungsgerät 100 zu verwendenden Betriebsparameters des Blutbehandlungsgeräts 100 ermöglicht und/oder gestartet werden, wenn die erfasste, zumindest eine Eigenschaft des zumindest einen zu verwendenden Einwegartikels 20, 21, 22, 23 in der Zuordnungsvorschrift dem erfassten, zumindest einen voreingestellten Wert des zumindest einen bei der Durchführung der Blutbehandlung zu verwendenden Betriebsparameters zugeordnet ist, wobei die Durchführung der Blutbehandlung mit dem Blutbehandlungsgerät 100 mit dem erfassten zumindest einen voreingestellten Wert des zumindest einen bei der Durchführung der Blutbehandlung von dem Blutbehandlungsgerät 100 zu verwendenden Betriebsparameters des Blutbehandlungsgeräts 100 blockiert wird, wenn die erfasste, zumindest eine Eigenschaft des zumindest einen zu verwendenden Einwegartikels 20, 21, 22, 23 in der Zuordnungsvorschrift dem erfassten, zumindest einen voreingestellten Wert des zumindest einen bei der Durchführung der Blutbehandlung zu verwendenden Betriebsparameters nicht zugeordnet ist.

Hierbei kann die Durchführung der Blutbehandlung mit dem Blutbehandlungsgerät 100 ermöglicht und/oder gestartet werden, wenn eine Bestätigung einer mittels der Ein- und Ausgabeeinrichtung 11 eingegebenen Eingabe der erfassten, zumindest einen Eigenschaft des zumindest einen bei der Durchführung der Blutbehandlung zu verwendenden Einwegartikels 20, 21, 22, 23 an der Ein- und Ausgabeeinrichtung 11 erfasst wird, wobei die Durchführung der Blutbehandlung mit dem Blutbehandlungsgerät 100 blockiert wird, indem die Bestätigung der mittels der Ein- und Ausgabeeinrichtung 11 eingegebenen Eingabe der erfassten, zumindest einen Eigenschaft des zumindest einen bei der Durchführung der Blutbehandlung zu verwendenden Einwegartikels 20, 21, 22, 23 an der Ein- und Ausgabeeinrichtung 11 blockiert wird, und optional zusätzlich eine optische und/oder akustische Warnmeldung, in einigen Ausführungen von der Steuereinrichtung 10, generiert und mittels der Ein- und Ausgabeeinrichtung 11 ausgegeben wird, wenn die Durchführung der Blutbehandlung mit dem Blutbehandlungsgerät 100 blockiert wird und/oder basierend auf der Zuordnungsvorschrift eine optische und/oder akustische Mitteilung mit einer Empfehlung zur Einstellung des zumindest einen bei der Durchführung der Blutbehandlung zu verwendenden Betriebsparameters, insbesondere von der Steuereinrichtung, generiert wird und mittels der Ein- und Ausgabeeinrichtung 11 ausgegeben wird.

In einigen Ausführungen wird der zumindest eine voreingestellte Wert des zumindest einen bei der Durchführung der Blutreinigung von dem Blutbehandlungsgerät 100 zu verwendenden Betriebsparameters des Blutbehandlungsgeräts 100 erfasst, wobei eine Eingabe, unter Verwendung der Ein- und Ausgabeeinrichtung 11, der zumindest einen Eigenschaft des zumindest einen bei der Durchführung der Blutbehandlung zu verwendenden Einwegartikels 20, 21, 22, 23 nur ermöglicht wird, wenn die zumindest eine Eigenschaft des zumindest einen Einwegartikels 20, 21, 22, 23 in der Zuordnungsvorschrift dem zumindest einen voreingestellten Wert des zumindest einen bei der Durchführung der Blutbehandlung von dem Blutbehandlungsgerät 100 zu verwendenden Betriebsparameters des Blutbehandlungsgeräts 100 zugeordnet ist, wobei die Durchführung der Blutbehandlung mit dem Blutbehandlungsgerät 100 mit dem erfassten, zumindest einen voreingestellten Wert des zumindest einen bei der Durchführung der Blutbehandlung von dem Blutbehandlungsgerät 100 zu verwendenden Betriebsparameters des Blutbehandlungsgeräts 100 ermöglicht und/oder gestartet wird, wenn eine Eingabe der zumindest einen Eigenschaft des zumindest einen zu verwendenden Einwegartikels 20, 21, 22, 23, die in der Zuordnungsvorschrift dem zumindest einen voreingestellten Wert des bei der Durchführung der Blutbehandlung zu verwendenden zumindest einen Betriebsparameters zugeordnet ist, erfasst wird.

In einigen Ausführungen wird die zumindest eine Eigenschaft des zumindest einen bei der Durchführung der Blutbehandlung zu verwendenden Einwegartikels 20, 21, 22, 23 erfasst, wobei zumindest ein Wert des zumindest einen bei der Durchführung der Blutbehandlung von dem Blutbehandlungsgerät 100 zu verwendenden Betriebsparameters erfasst wird, und die Durchführung der Blutbehandlung mit dem Blutbehandlungsgerät 100 oder eine Fortsetzung einer momentan mit dem Blutbehandlungsgerät 100 durchgeführten Blutbehandlung mit dem erfassten zumindest einen Wert des zumindest einen bei der Durchführung der Blutbehandlung von dem Blutbehandlungsgerät 100 zu verwendenden Betriebsparameters des Blutbehandlungsgeräts 100 ermöglicht und/oder gestartet wird, wenn die erfasste, zumindest eine Eigenschaft des zumindest einen zu verwendenden Einwegartikels 20, 21, 22, 23 in der Zuordnungsvorschrift dem erfassten, zumindest einen Wert des zumindest einen bei der Durchführung der Blutbehandlung von dem Blutbehandlungsgerät 100 zu verwendenden Betriebsparameters zugeordnet ist, und die Durchführung der Blutbehandlung mit dem Blutbehandlungsgerät 100 oder die Fortsetzung der momentan mit dem Blutbehandlungsgerät 100 durchgeführten Blutbehandlung mit dem erfassten zumindest einen Wert des zumindest einen bei der Durchführung der Blutbehandlung von dem Blutbehandlungsgerät 100 zu verwendenden Betriebsparameters des Blutbehandlungsgeräts 100 blockiert wird, wenn die erfasste, zumindest eine Eigenschaft des zumindest einen bei der Durchführung der Blutbehandlung zu verwendenden Einwegartikels 20, 21, 22, 23 in der Zuordnungsvorschrift dem erfassten, zumindest einen Wert des bei der Durchführung der Blutbehandlung zu verwendenden zumindest einen Betriebsparameters nicht zugeordnet ist.

Hierbei kann die Durchführung der Blutbehandlung mit dem Blutbehandlungsgerät 100 oder die Fortsetzung der momentan mit dem Blutbehandlungsgerät 100 durchgeführten Blutbehandlung ermöglicht und/oder gestartet werden, wenn eine Bestätigung einer mittels der Ein- und Ausgabeeinrichtung 11 eingegebenen Eingabe der erfassten, zumindest einen Eigenschaft des zumindest einen bei der Durchführung der Blutbehandlung zu verwendenden Einwegartikels 20, 21, 22, 23 an der Ein- und Ausgabeeinrichtung 11 erfasst wird, und die Durchführung der Blutbehandlung mit dem Blutbehandlungsgerät 100 oder die Fortsetzung der momentan mit dem Blutbehandlungsgerät 100 durchgeführten Blutbehandlung blockiert werden, indem die Bestätigung der mittels der Ein- und Ausgabeeinrichtung 11 eingegebenen Eingabe der erfassten, zumindest einen Eigenschaft des zumindest einen zu verwendenden Einwegartikels 20, 21, 22, 23 an der Ein- und Ausgabeeinrichtung blockiert wird.

Das Erfassen der zumindest einen Eigenschaft des zumindest einen bei der Durchführung der Blutbehandlung zu verwendenden Einwegartikels 20, 21, 22, 23 kann ein Erfassen einer Eingabe der zumindest einen Eigenschaft des zumindest einen bei der Durchführung der Blutbehandlung zu verwendenden Einwegartikels 20, 21, 22, 23 mittels einer grafischen Benutzeroberfläche der Ein- und Ausgabeeinrichtung 11 und/oder ein Herunterladen der zumindest einen Eigenschaft des zumindest einen bei der Durchführung der Blutbehandlung zu verwendenden Einwegartikels 20, 21, 22, 23 mittels der Ein- und Ausgabeeinrichtung 11 von einem externen Gerät 40 und/oder ein Scannen eines dem zumindest einen bei der Durchführung der Blutbehandlung zu verwendenden Einwegartikel zugeordneten Codes mittels einer Scan-Einrichtung der Ein- und Ausgabeeinrichtung 11, wobei die Steuereinrichtung 10 die zumindest eine Eigenschaft des zumindest einen bei der Durchführung der Blutbehandlung zu verwendenden Einwegartikels 20, 21, 22, 23 basierend auf dem gescannten Code erfasst, beinhalten.

## Patentansprüche

1. Verfahren zur Steuerung eines Blutbehandlungsgeräts (100), insbesondere Dialysegeräts, das eine Steuereinrichtung (10) und eine als Ein- und Ausgabeeinrichtung (11) ausgebildete Komponente des Blutbehandlungsgeräts (100) aufweist, und das dazu eingerichtet ist, gesteuert von der Steuereinrichtung (10), eine Blutbehandlung unter Verwendung zumindest eines Einwegartikels (20, 21, 22, 23) und zumindest eines Werts zumindest eines Betriebsparameters des Blutbehandlungsgeräts (100) zumindest teilweise automatisiert durchzuführen, wobei das Verfahren die folgenden Schritte aufweist:
Erfassen, insbesondere mittels der Ein- und Ausgabeeinrichtung (11), einer Zuordnungsvorschrift, in der zumindest eine Eigenschaft, insbesondere eine physikalische Eigenschaft, zumindest eines zur Blutbehandlung verwendbaren Einwegartikels (20, 21, 22, 23) zumindest einem Wert eines bei der Durchführung der Blutbehandlung verwendbaren zumindest einen Betriebsparameters, insbesondere des Blutbehandlungsgeräts, zugeordnet ist, und
Steuern, mittels der Steuereinrichtung (10), zumindest einer Komponente (11, 14), insbesondere der Ein- und Ausgabeeinrichtung (11), des Blutbehandlungsgeräts (100), basierend auf der Zuordnungsvorschrift.

2. Verfahren nach Anspruch 1, ferner aufweisend:
Erfassen zumindest eines, insbesondere in einer Speichereinrichtung (12) des Blutbehandlungsgeräts (100) gespeicherten, voreingestellten Werts zumindest eines bei der Durchführung der Blutbehandlung von dem Blutbehandlungsgerät (100) zu verwendenden Betriebsparameters des Blutbehandlungsgeräts (100), und/oder
Erfassen zumindest einer Eigenschaft, insbesondere einer physikalischen Eigenschaft, zumindest eines bei der Durchführung der Blutbehandlung zu verwendenden Einwegartikels (20, 21, 22, 23), wobei
das Steuern der zumindest einen Komponente (11, 14) des Blutbehandlungsgeräts (100) ferner basierend
auf dem zumindest einen voreingestellten Wert des zumindest einen bei der Durchführung der Blutbehandlung von dem Blutbehandlungsgerät (100) zu verwendenden Betriebsparameters des Blutbehandlungsgeräts (100), und/oder
auf der erfassten, zumindest einen Eigenschaft des zumindest einen bei der Durchführung der Blutbehandlung zu verwendenden Einwegartikels (20, 21, 22, 23)
erfolgt.

3. Verfahren nach Anspruch 2, wobei
der zumindest eine voreingestellte Wert des zumindest einen bei der Durchführung der Blutbehandlung von dem Blutbehandlungsgerät (100) zu verwendenden Betriebsparameters des Blutbehandlungsgeräts (100) erfasst wird, und
die zumindest eine Eigenschaft des bei der Durchführung der Blutbehandlung zumindest einen zu verwendenden Einwegartikels (20, 21, 22, 23) erfasst wird, und das Verfahren ferner aufweist:
Durchführen einer Prüfung, ob die erfasste, zumindest eine Eigenschaft des zumindest einen bei der Durchführung der Blutbehandlung zu verwendenden Einwegartikels (20, 21, 22, 23) in der Zuordnungsvorschrift dem zumindest einen voreingestellten Wert des bei der Durchführung der Blutbehandlung zu verwendenden zumindest einen Betriebsparameters zugeordnet ist oder nicht, wobei
das Steuern der zumindest einen Komponente (11, 14) des Blutbehandlungsgeräts (100) ferner basierend auf einem Ergebnis der durchgeführten Prüfung erfolgt.

4. Verfahren nach Anspruch 3, wobei
die Durchführung der Blutbehandlung mit dem Blutbehandlungsgerät (100) mit dem erfassten zumindest einen voreingestellten Wert des zumindest einen bei der Durchführung der Blutbehandlung von dem Blutbehandlungsgerät (100) zu verwendenden Betriebsparameters des Blutbehandlungsgeräts (100) ermöglicht und/oder gestartet wird, wenn die erfasste, zumindest eine Eigenschaft des zumindest einen zu verwendenden Einwegartikels (20, 21, 22, 23) in der Zuordnungsvorschrift dem erfassten, zumindest einen voreingestellten Wert des zumindest einen bei der Durchführung der Blutbehandlung zu verwendenden Betriebsparameters zugeordnet ist, und
die Durchführung der Blutbehandlung mit dem Blutbehandlungsgerät (100) mit dem erfassten zumindest einen voreingestellten Wert des zumindest einen bei der Durchführung der Blutbehandlung von dem Blutbehandlungsgerät (100) zu verwendenden Betriebsparameters des Blutbehandlungsgeräts (100) blockiert wird, wenn die erfasste, zumindest eine Eigenschaft des zumindest einen zu verwendenden Einwegartikels (20, 21, 22, 23) in der Zuordnungsvorschrift dem erfassten, zumindest einen voreingestellten Wert des zumindest einen bei der Durchführung der Blutbehandlung zu verwendenden Betriebsparameters nicht zugeordnet ist.

5. Verfahren nach Anspruch 4, wobei
die Durchführung der Blutbehandlung mit dem Blutbehandlungsgerät (100) ermöglicht und/oder gestartet wird, wenn eine Bestätigung einer mittels der Ein- und Ausgabeeinrichtung (11) eingegebenen Eingabe der erfassten, zumindest einen Eigenschaft des zumindest einen bei der Durchführung der Blutbehandlung zu verwendenden Einwegartikels (20, 21, 22, 23) an der Ein- und Ausgabeeinrichtung (11) erfasst wird, und
die Durchführung der Blutbehandlung mit dem Blutbehandlungsgerät (100) blockiert wird, indem die Bestätigung der mittels der Ein- und Ausgabeeinrichtung (11) eingegebenen Eingabe der erfassten, zumindest einen Eigenschaft des zumindest einen bei der Durchführung der Blutbehandlung zu verwendenden Einwegartikels (20, 21, 22, 23) an der Ein- und Ausgabeeinrichtung (11) blockiert wird, wobei
optional zusätzlich eine optische und/oder akustische Warnmeldung, insbesondere von Steuereinrichtung (10), generiert und mittels der Ein- und Ausgabeeinrichtung (11) ausgegeben wird, wenn die Durchführung der Blutbehandlung mit dem Blutbehandlungsgerät (100) blockiert wird und/oder basierend auf der Zuordnungsvorschrift eine optische und/oder akustische Mitteilung mit einer Empfehlung zur Einstellung des zumindest einen bei der Durchführung der Blutbehandlung zu verwendenden Betriebsparameters, insbesondere von der Steuereinrichtung, generiert wird und mittels der Ein- und Ausgabeeinrichtung (11) ausgegeben wird.

6. Verfahren nach Anspruch 2, wobei
der zumindest eine voreingestellte Wert des zumindest einen bei der Durchführung der Blutreinigung von dem Blutbehandlungsgerät (100) zu verwendenden Betriebsparameters des Blutbehandlungsgeräts (100) erfasst wird, und
eine Eingabe, insbesondere unter Verwendung der Ein- und Ausgabeeinrichtung (11), der zumindest einen Eigenschaft des zumindest einen bei der Durchführung der Blutbehandlung zu verwendenden Einwegartikels (20, 21, 22, 23) nur ermöglicht wird, wenn die zumindest eine Eigenschaft des zumindest einen Einwegartikels (20, 21, 22, 23) in der Zuordnungsvorschrift dem zumindest einen voreingestellten Wert des zumindest einen bei der Durchführung der Blutbehandlung von dem Blutbehandlungsgerät (100) zu verwendenden Betriebsparameters des Blutbehandlungsgeräts (100) zugeordnet ist, wobei
die Durchführung der Blutbehandlung mit dem Blutbehandlungsgerät (100) mit dem erfassten, zumindest einen voreingestellten Wert des zumindest einen bei der Durchführung der Blutbehandlung von dem Blutbehandlungsgerät (100) zu verwendenden Betriebsparameters des Blutbehandlungsgeräts (100) ermöglicht und/oder gestartet wird, wenn eine Eingabe der zumindest einen Eigenschaft des zumindest einen zu verwendenden Einwegartikels (20, 21, 22, 23), die in der Zuordnungsvorschrift dem zumindest einen voreingestellten Wert des bei der Durchführung der Blutbehandlung zu verwendenden zumindest einen Betriebsparameters zugeordnet ist, erfasst wird.

7. Verfahren nach einem der Ansprüche 2 bis 6, wobei
die zumindest eine Eigenschaft des zumindest einen bei der Durchführung der Blutbehandlung zu verwendenden Einwegartikels (20, 21, 22, 23) erfasst wird, und
zumindest ein Wert des zumindest einen bei der Durchführung der Blutbehandlung von dem Blutbehandlungsgerät (100) zu verwendenden Betriebsparameters erfasst wird, wobei
die Durchführung der Blutbehandlung mit dem Blutbehandlungsgerät (100) oder eine Fortsetzung einer momentan mit dem Blutbehandlungsgerät (100) durchgeführten Blutbehandlung mit dem erfassten zumindest einen Wert des zumindest einen bei der Durchführung der Blutbehandlung von dem Blutbehandlungsgerät (100) zu verwendenden Betriebsparameters des Blutbehandlungsgeräts (100) ermöglicht und/oder gestartet wird, wenn die erfasste, zumindest eine Eigenschaft des zumindest einen zu verwendenden Einwegartikels (20, 21, 22, 23) in der Zuordnungsvorschrift dem erfassten, zumindest einen Wert des zumindest einen bei der Durchführung der Blutbehandlung von dem Blutbehandlungsgerät (100) zu verwendenden Betriebsparameters zugeordnet ist, und
die Durchführung der Blutbehandlung mit dem Blutbehandlungsgerät (100) oder die Fortsetzung der momentan mit dem Blutbehandlungsgerät (100) durchgeführten Blutbehandlung mit dem erfassten zumindest einen Wert des zumindest einen bei der Durchführung der Blutbehandlung von dem Blutbehandlungsgerät (100) zu verwendenden Betriebsparameters des Blutbehandlungsgeräts (100) blockiert wird, wenn die erfasste, zumindest eine Eigenschaft des zumindest einen bei der Durchführung der Blutbehandlung zu verwendenden Einwegartikels (20, 21, 22, 23) in der Zuordnungsvorschrift dem erfassten, zumindest einen Wert des bei der Durchführung der Blutbehandlung zu verwendenden zumindest einen Betriebsparameters nicht zugeordnet ist.

8. Verfahren nach Anspruch 7, wobei
die Durchführung der Blutbehandlung mit dem Blutbehandlungsgerät (100) oder die Fortsetzung der momentan mit dem Blutbehandlungsgerät (100) durchgeführten Blutbehandlung ermöglicht und/oder gestartet wird, wenn eine Bestätigung einer mittels der Ein- und Ausgabeeinrichtung (11) eingegebenen Eingabe der erfassten, zumindest einen Eigenschaft des zumindest einen bei der Durchführung der Blutbehandlung zu verwendenden Einwegartikels (20, 21, 22, 23) an der Ein- und Ausgabeeinrichtung (11) erfasst wird, und
die Durchführung der Blutbehandlung mit dem Blutbehandlungsgerät (100) oder die Fortsetzung der momentan mit dem Blutbehandlungsgerät (100) durchgeführten Blutbehandlung blockiert wird, indem die Bestätigung der mittels der Ein- und Ausgabeeinrichtung (11) eingegebenen Eingabe der erfassten, zumindest einen Eigenschaft des zumindest einen zu verwendenden Einwegartikels (20, 21, 22, 23) an der Ein- und Ausgabeeinrichtung blockiert wird.

9. Verfahren nach einem der Ansprüche 2 bis 8, wobei das Erfassen der zumindest einen Eigenschaft des zumindest einen bei der Durchführung der Blutbehandlung zu verwendenden Einwegartikels (20, 21, 22, 23) ein Erfassen einer Eingabe der zumindest einen Eigenschaft des zumindest einen bei der Durchführung der Blutbehandlung zu verwendenden Einwegartikels (20, 21, 22, 23) mittels einer grafischen Benutzeroberfläche der Ein- und Ausgabeeinrichtung (11) und/oder ein Herunterladen der zumindest einen Eigenschaft des zumindest einen bei der Durchführung der Blutbehandlung zu verwendenden Einwegartikels (20, 21, 22, 23) mittels der Ein- und Ausgabeeinrichtung (11) von einem externen Gerät (40) und/oder ein Scannen eines dem zumindest einen bei der Durchführung der Blutbehandlung zu verwendenden Einwegartikel zugeordneten Codes mittels einer Scan-Einrichtung der Ein- und Ausgabeeinrichtung (11), wobei die Steuereinrichtung (10) die zumindest eine Eigenschaft des zumindest einen bei der Durchführung der Blutbehandlung zu verwendenden Einwegartikels (20, 21, 22, 23) basierend auf dem gescannten Code erfasst, beinhaltet.

10. Verfahren nach einem der vorhergehenden Ansprüche, wobei jeweilige Werte des zumindest einen Werts eines Betriebsparameters des zumindest einen Betriebsparameters jeweiligen unterschiedlichen Arten der Blutbehandlung zugeordnet sind.

11. Verfahren nach einem der vorhergehenden Ansprüche, wobei der zumindest eine Einwegartikel (20, 21, 22, 23) einen Dialysator (20), ein Blutschlauchsystem (21), eine venöse Nadel (22), und ein oder mehrere Dialysekonzentrate (23) beinhaltet, die zumindest eine Eigenschaft des Dialysators (20) einen Ultrafiltrationskoeffizienten des Dialysators (20), ein Volumen des Dialysators (20), und einen Längswiderstand des Dialysators (20) beinhaltet, die zumindest eine Eigenschaft des Blutschlauchsystems (21) ein Volumen des Blutschlauchsystems (21), einen Flusswiderstand des Blutschlauchsystems (21) und eine Eignung des Blutschlauchsystems (21) für Pre-, Post- und/oder Mixed-Substitution im Rahmen eines Hämodiafiltration-Substitutionsverfahrens, und die zumindest eine Eigenschaft der venösen Nadel (22) einen Flusswiderstand der venösen Nadel (22) beinhaltet, wobei das Blutbehandlungsgerät (100) als eine weitere Komponente eine Blutpumpe (14) aufweist, die während der Durchführung der Blutbehandlung über das Blutschlauchsystem (21) mit dem Dialysator (20) und der venösen Nadel (22) fluidisch verbunden ist, und wobei der zumindest eine Wert zumindest eines Betriebsparameters des Blutbehandlungsgeräts (100) einen Wert einer Förderrate der Blutpumpe (14) beinhaltet.

12. Vorrichtung (200), die dazu eingerichtet ist, ein Verfahren zur Steuerung eines Blutbehandlungsgeräts (100), insbesondere Dialysegeräts, nach einem der vorhergehenden Ansprüche durchzuführen.
